(19) Europäisches Patentamt
European Patent Office
Office européen des brevets

(11) Veröffentlichungsnummer: **0 106 805**
**B1**

# (12) EUROPÄISCHE PATENTSCHRIFT

(45) Veröffentlichungstag der Patentschrift: **27.08.86**

(21) Anmeldenummer: **83810459.4**

(22) Anmeldetag: **06.10.83**

(51) Int. Cl.[4]: **C 07 D 495/06**, C 07 D 517/06 // (C07D495/06, 339:00, 339:00),(C07D517/06, 345:00, 345:00)

## (54) Verfahren zur Herstellung von 3,4,9,10-Tetrathioperylen und 3,4,9,10-Tetraselenoperylen.

(30) Priorität: **12.10.82 CH 5962/82**

(43) Veröffentlichungstag der Anmeldung: **25.04.84 Patentblatt 84/17**

(45) Bekanntmachung des Hinweises auf die Patenterteilung: **27.08.86 Patentblatt 86/35**

(84) Benannte Vertragsstaaten: **BE CH DE FR GB LI NL**

(56) Entgegenhaltungen: **SOLID STATE COMMUNICATIONS, Band 38, Nr. 12, 1981, Seiten 1129-1134, Pergamon Press Ltd., GB. B. HILTI et al.: "3,4,9,10 - Tetrathioperylene - I 1.28' A new highly conducting, incommensurate organic charge transfer salt"**

(73) Patentinhaber: **CIBA-GEIGY AG, Klybeckstrasse 141, CH-4002 Basel (CH)**

(72) Erfinder: **Mayer, Carl Walter, Dr., Niederholzboden 55, CH-4125 Riehen (CH)**

**EP 0 106 805 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von 3,4,9,10-Tetrathioperylen und 3,4,9,10-Tetraselenoperylen.

Es ist bekannt, dass polycyclische Aromaten, die in den Peristellungen durch Chalkogenbrükken modifiziert sind, wertvollere Donoren für organische Leiter oder Halbleiter darstellen; vgl. z.B. deutsche Patentschrift 2 641 742 und U.S. Patentschrift 3 984 593; J. Am. Chem. Soc., 98 : 1,252 (1976) und 99 : 1,255 (1977); J. Org. Chem., 30, 3997 (1965) und U.S. Patentschriften 3 403 165 und 3 634 366. In der letztgenannten U.S. Patentschrift werden unter einer Vielzahl von mit Chalkogenbrücken modifizierten polycyclischen Aromaten auch Tetrachalkogenperylene, wie 3,4,9,10-Tetrathio-, 3,4,9,10-Tetratellur- und 3,4,9,10-Tetraselenopyerylen genannt. Nach dem aus Organometallics, 1, 739 (1982) bekannten Verfahren zur Herstellung von 5,6,11,12-Tetratellurotetracen durch Umsetzung von 5,6,11,12-Tetrachlortetracen mit Natriumditellurid in Gegenwart von N,N-Dimethylformamid oder Hexamethylphosporamid lassen sich 3,4,9,10-Tetrathio-, 3,4,9,10-Tetratellur- oder 3,4,9,10-Tetraselenoperylen nicht in reiner Form und/oder nur in Spuren herstellen. Auch die Reaktion mit Natriumdiselenid führt zu Produkten, die noch Chlor enthalten (J. Veigl, Dissertation, Universität Heidelberg 1981).

Unsubstituiertes Perylen oder 1,12- und 3,10-Dihydroxyperylen können z.B. durch Erhitzen von Naphthalin oder Naphthalinderivaten, wie 1-Bromnaphthalin, 1,1'-Binaphthyl, 1,1'- oder 2,2'-Binaphthol, in Gegenwart von Aluminiumchlorid auf etwa 150–160 °C hergestellt werden. Die Ausbeuten sind jedoch im allgemeinen sehr gering. Unsubstituiertes Perylen kann auch durch Cyclisierung von 1,1'-Binaphthyl in Gegenwart von mindestens 36%iger Fluorwasserstoffsäure und Mangandioxid bei ca. 140 °C, durch Erhitzen von 2,2'-Binaphthol mit Phosphorpentachlorid und Phosphorsäure auf 400–500 °C oder mit Phosphoroxichlorid und Zinkstaub auf 500–600 °C oder durch Destillation von 1,12- oder 3,10-Dihydroxyperylen mit Zinkstaub erhalten werden. Bei diesen vorbekannten Verfahren müssen somit recht drastische Reaktionsbedingungen angewendet werden, denen verschiedene Substituenten an den Naphthalinen oder Naphthalinderivaten, u.a. Chalkogenbrücken, nicht standhalten. Vgl. z.B. E. Clar, Polycyclic Hydrocarbons, Bd. 2, S. 24 ff., Academic Press (1964).

Andererseits ist bekannt, dass nach der sog. Schollschen Reaktion durch gelindes Erwärmen von 1-Methoxy- oder 1-Äthoxynaphthalin in Gegenwart von Nitrobenzol und einer Lewis- oder Protonensäure, besonders Aluminiumchlorid oder Benzolsulfonsäure, 1,1'-Dimethoxy- oder 1,1'-Diäthoxy- 4,4'-binaphthyl entstehen. Verwendet man z.B. anstelle von 1-Methoxynaphthalin das 1,8-Dimethoxynaphthalin, so findet unter den genannten Reaktionsbedingungen ebenfalls nur eine einfache Verknüpfung zum 1,1',8,8'-Tetramethoxy-4,4'-binaphthyl statt [vgl. z.B. Chem. Ber. 55, 330 (1922) und Chem. Ber. 91, 2109 (1958)].

Es wurde nun gefunden, dass sich die chalkogensubstituierten Perylene der Formel I

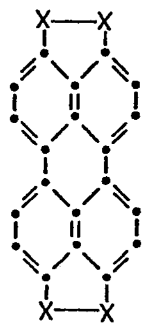

(I),

worin X S oder Se bedeutet, überraschenderweise auf einfache Weise und unter milden Reaktionsbedingungen in reiner Form dadurch herstellen lassen, dass man eine Verbindung der Formel II

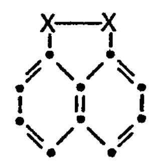

(II),

worin X die unter Formel I angegebene Bedeutung hat, in Nitrobenzol oder einem Gemisch aus Nitrobenzol und einem damit mischbaren, unter den Reaktionsbedingungen inerten organischen Lösungsmittel in Gegenwart einer Lewis-Säure oder einer Protonensäure auf 20 bis 120 °C erhitzt.

Für die Umsetzung der Verbindung der Formel II, worin X S darstellt, verwendet man bevorzugt eine Lewis-Säure und die Reaktionstemperaturen liegen zweckmässig zwischen 20 und 40 °C. Für die Umsetzung der Verbindung der Formel II, worin X Se ist, werden Protonensäuren und Reaktionstemperaturen zwischen 80 und 100 °C bevorzugt.

Als Lewis-Säuren kommen z.B. Aluminiumchlorid, Aluminiumtribromid, $BF_3$, Zinntetrachlorid, Zinkchlorid und Titantetrachlorid in Betracht. Bevorzugt verwendet man als Lewis-Säure Aluminiumtrichlorid.

Geeignete Protonensäuren sind z.B. Halogenwasserstoffsäuren, wie HF, HCl und HBr, Schwefelsäure, Phosphorsäure, Polyphosphorsäure oder gegebenenfalls halogenierte aliphatische Carbonsäuren, wie Dichloressigsäure und Trifluoressigsäure, vor allem jedoch aliphatische oder aromatische Sulfonsäuren, wie Methansulfonsäure, Benzolsulfonsäure, p-Toluolsulfonsäure oder Naphthalinsulfonsäure. Bevorzugt verwendet man als Protonensäure eine aromatische Sulfonsäure, besonders Benzolsulfonsäure oder p-Toluolsulfonsäure.

Als unter den Reaktionsbedingungen inerte, mit Nitrobenzol mischbare organische Lösungsmittel können beispielsweise aromatische Kohlenwasserstoffe, wie Benzol oder Toluol, verwendet werden. Werden Gemische aus Nitrobenzol und einem damit mischbaren inerten Lösungsmittel eingesetzt, so beträgt der Anteil an Nitrobenzol pro Mol Verbindung der Formel II zweckmässig mindestens 1/2 Mol. Bevorzugt wird die Umsetzung in reinem Nitrobenzol, besonders wasserfreiem Nitrobenzol, vorgenommen.

Die Aufarbeitung der Verbindungen der Formel I wird mit Vorteil in Gegenwart eines Reduktionsmittels, wie $TiCl_3$ durchgeführt, um allfällige Anteile an durch das Nitrobenzol oxidiertem Endprodukt in die gewünschte Form zu bringen. Die erfindungsgemäss erhaltenen Rohprodukte werden zweckmässig durch Sublimation gereinigt.

Die Ausgangsprodukte der Formel II sind bekannt [vgl. J. Am. Chem. Soc., 99 : 1, 255 (1977)].

Die Verbindungen der Formel I eignen sich – wie schon erwähnt – z.B. als Donoren zur Herstellung von organischen Leitern oder Halbleitern, wobei als Elektronenakzeptoren beispielsweise Benzochinone der in der U.S. Patentschrift 3 403 165 genannten Art, Halogene, wie Chlor, Brom und insbesondere Jod, $PF_6^-$, $AsF_6^-$, $SbF_6^-$, $TaF_6^-$, $ClO_4^-$, $ReO_4^-$ oder $FSO_3^-$, 7,7,8,8-Tetracyanochinodimethan oder organische Säuren, wie Carbonsäuren oder Sulfonsäuren, verwendet werden können (s. z.B. U.S. Patentschrift 3 634 336). Leitende Komplexe aus 3,4,9,10-Tetrathioperylen und Jod können z.B. durch Co-Sublimation von 3,4,9,10-Tetrathioperylen und Jod hergestellt werden [vgl. Solid State Comm., 38, 1129 (1981)].

Beispiel 1

In einem 350 ml Sulfierkolben werden unter Argon 6,9 g (51,74 mMol) Aluminiumchlorid in 200 ml trockenem Nitrobenzol gelöst. Die Lösung wird auf 10 °C abgekühlt. Dann gibt man eine Lösung von 7,6 g (40 mMol) Naphtho[1,8-c,d]-1,2-dithiol in 70 ml trockenem Nitrobenzol zu. Das entstandene dunkelblaue Gemisch wird anschliessend zunächst 1/2 Stunde bei 10 °C und dann 24 Stunden bei 40 °C gerührt. Dabei scheidet sich eine rotviolette Suspension ab. Anschliessend wird das Reaktionsgemisch auf 500 ml 1N HCl gegossen und gut durchgerührt. Man gibt 50 ml 15%iges $TiCl_3$ in 10%iger HCl zu und lässt eine Stunde ausrühren. Nach dem Abnutschen wird der erhaltene Festkörper gut mit Wasser und Diäthyläther gewaschen und dann bei 40 °C/0,13 Pa am Hochvakuum getrocknet. Man erhält 2,2 g (29% d.Th.) rohes Tetrathioperylen. Das Rohprodukt wird anschliessend bei 300 °C /0,13 Pa sublimiert, wobei sich das reine Tetrathioperylen in goldenen, glänzenden Nädelchen abscheidet (1,2 g; ca. 16% d.Th.). Identifikation durch Massenspektrum: $M^+=376$. Vis-Spektrum in 1,2,4-Trichlorbenzol: $\lambda_{max}$ 572 nm und 531 nm. Kristallstruktur gemäss X-ray: monoklin, Raumgruppe $P2_1/n$ (zentrosymmetrisch); Achsen: a = 16,149 Å, b : 4,013 Å, c = 22,292 Å, $\beta$ = 94,54°

Beispiel 2

In einem 250 ml Dreihalskolben wird unter Argon ein Gemisch von 1,0 g (3,49 mMol) Naphtho[1,8-c,d]-1,2-diselenol und 5,0 g (31,6 mMol) wasserfreie Benzolsulfonsäure in 100 ml trockenem Nitrobenzol während 20 Stunden bei 100 °C gerührt. Darauf wird die rotviolette Lösung am Hochvakuum eingedampft. Nach dem Trocknen über Nacht bei 60 °C am Hochvakuum versetzt man den öligen Rückstand mit ca. 250 ml 10%iger Natriumbicarbonatlösung. Der dabei entstehende kristalline Niederschlag wird abgesaugt, wiederholt mit Natriumbicarbonatlösung und anschliessend mit 1N HCl-Lösung gewaschen und während 30 Minuten mit 15%igem $TiCl_3$ in 10%iger HCl (20 ml) verrührt. Anschliessend wird das Produkt mit Wasser neutral gewaschen und am Hochvakuum getrocknet. Man erhält 1 g (100% d.Th.) Rohprodukt. Das Rohprodukt wird bei 375 °C /0,13 Pa sublimiert, wodurch man 110 mg (11% d.Th.) reines Tetraselenoperylen in Form silbrig glänzender Nädelchen erhält. Identifikation durch Massenspektrum: $M^+= 564$, Isotopencluster entspricht 4 Se-Atomen pro Molekül. Vis-Spektrum in Benzol: $\lambda_{max}$ 574 nm, 532 nm.

Kristallstruktur gemäss X-ray: monoklin, Raumgruppe $P2_1/c$; Achsen a = 7,896 Å, c = 22,578 Å, $\beta$ = 90,57°.

**Patentansprüche**

1. Verfahren zur Herstellung von chalkogen-substituierten Perylenen der Formel I

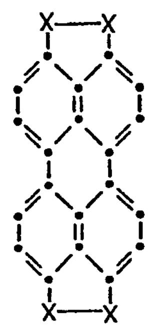

(I),

worin X S oder Se bedeutet, dadurch gekennzeichnet, dass man eine Verbindung der Formel II

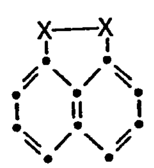

(II),

worin X die unter Formel I angegebene Bedeutung hat, in Nitrobenzol oder einem Gemisch aus Nitrobenzol und einem damit mischbaren, unter den Reaktionsbedingungen inerten organischen Lösungsmittel in Gegenwart einer Lewis-Säure oder einer Protonensäure auf 20 bis 120 °C erhitzt.

2. Verfahren nach Anspruch 1, dadurch gekennzeichnet, dass man die Verbindung der Formel II, worin X S darstellt, in Gegenwart einer Lewis-Säure auf 20 bis 40 °C erhitzt.

3. Verfahren nach Anspruch 2, dadurch gekennzeichnet, dass man die Verbindung der Formel II, worin X S darstellt, in Gegenwart von Aluminiumchlorid auf 20 bis 40 °C erhitzt.

4. Verfahren nach Anspruch 2, dadurch gekennzeichnet, dass man die Verbindung der Formel II, worin X Se darstellt, in Gegenwart einer Protonensäure auf 80 bis 100 °C erhitzt.

5. Verfahren nach Anspruch 2, dadurch gekennzeichnet, dass man die Verbindung der Formel II, worin X Se darstellt, in Gegenwart einer aromatischen Sulfonsäure auf 80 bis 100 °C erhitzt.

6. Verfahren nach Anspruch 2, dadurch gekennzeichnet, dass man die Verbindung der Formel II, worin X Se darstellt, in Gegenwart von Benzolsulfonsäure oder p-Toluolsulfonsäure auf 80 bis 100 °C erhitzt.

7. Verfahren nach Anspruch 1, dadurch gekennzeichnet, dass man die Umsetzung in reinem Nitrobenzol, insbesondere wasserfreiem Nitrobenzol, durchführt.

**Revendications**

1. Procédé pour préparer des pérylènes chalcogénés répondant à la formule I:

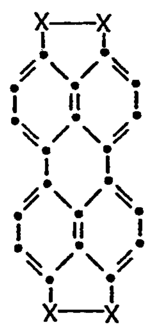

(I),

dans laquelle X représente S ou Se, procédé caractérisé en ce qu'on chauffe à une température de 20 à 120 °C un composé répondant à la formule II:

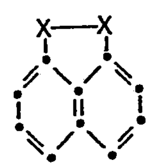

(II),

dans laquelle X a la signification indiquée au-dessous de la formule I, dans du nitrobenzène ou dans un mélange de nitrobenzène et d'un solvant organique miscible à celui-ci et inerte dans les conditions de la réaction, en présence d'un acide de Lewis ou d'un acide protonique.

2. Procédé selon la revendication 1, caractérisé en ce qu'on chauffe le composé de formule II dans lequel X représente S, à une température de 20 à 40 °C, en présence d'un acide de Lewis.

3. Procédé selon la revendication 2, caractérisé en ce qu'on chauffe le composé de formule II dans lequel X représente S, à une température de 20 à 40 °C, en présence de chlorure d'aluminium.

4. Procédé selon la revendication 1, caractérisé en ce qu'on chauffe le composé de formule II dans lequel X représente Se, à une température de 80 à 100 °C, en présence d'un acide protonique.

5. Procédé selon la revendication 4, caractérisé en ce qu'on chauffe le composé de formule II dans lequel X représente Se, à une température de 80 à 100 °C, en présence d'un acide sulfonique aromatique.

6. Procédé selon la revendication 5, caractérisé en ce qu'on chauffe le composé de formule II dans lequel X représente Se, à une température de 80 à 100 °C, en présence d'acide benzène-sulfonique ou d'acide p-toluène-sulfonique.

7. Procédé selon la revendication 1, caractérisé en ce qu'on effectue la réaction dans du nitrobenzène pur, plus spécialement dans du nitrobenzène anhydre.

**Claims**

1. A process for the preparation of a chalcogen-substituted perylene of the formula I

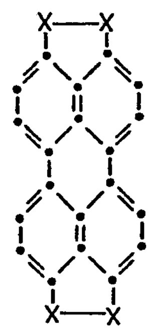

(I),

wherein X is S or Se, which process comprises heating a compound of the formula II

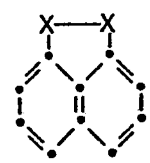

(II),

wherein X is as defined for the formula I, in nitrobenzene, or in a mixture of nitrobenzene and an organic solvent miscible therewith and inert under the reaction conditions, in the presence of a Lewis acid or a protonic acid to a temperature in the range from 20 to 120 °C.

2. A process according to claim 1, wherein the compound of the formula II wherein X is S is heated in the presence of a Lewis acid to a temperature in the range from 20 to 40 °C.

3. A process according to claim 2, wherein the compound of the formula II wherein X is S is heated in the presence of aluminium chloride to a temperature in the range from 20 to 40 °C.

4. A process according to claim 1, wherein the compound of the formula II wherein X is Se is heated in the presence of a protonic acid to a temperature in the range from 80 to 100 °C.

5. A process according to claim 4, wherein the compound of the formula II wherein X is Se is

heated in the presence of an aromatic sulfonic acid to a temperature in the range from 80 to 100 °C.

6. A process according to claim 5, wherein the compound of the formula II wherein X is Se is heated in the presence of benzenesulfonic acid or p-toluenesulfonic acid to a temperature in the range from 80 to 100 °C.

7. A process according to claim 1, wherein the reaction is performed in pure nitrobenzene, particularly in anhydrous nitrobenzene.